Europäisches Patentamt

European Patent Office

Office européen des brevets

(19)

(11) **EP 1 439 402 A1**

(12) **EUROPEAN PATENT APPLICATION**

| | |
|---|---|
| (43) Date of publication:<br> **21.07.2004 Bulletin 2004/30** | (51) Int Cl.7: **G01S 15/89**, A61B 8/08,<br> **G01N 29/06** |
| (21) Application number: **04000506.8** | |
| (22) Date of filing: **13.01.2004** | |

| | |
|---|---|
| (84) Designated Contracting States:<br> **AT BE BG CH CY CZ DE DK EE ES FI FR GB GR HU IE IT LI LU MC NL PT RO SE SI SK TR**<br> Designated Extension States:<br> **AL LT LV MK**<br><br>(30) Priority: **16.01.2003 JP 2003008749**<br><br>(71) Applicant: **MATSUSHITA ELECTRIC INDUSTRIAL CO., LTD.**<br> **Kadoma-shi, Osaka 571-8501 (JP)** | (72) Inventor: **Yoden, Sadato**<br> **Kadoma-shi Osaka, 571-0065 (JP)**<br><br>(74) Representative: **Balsters, Robert et al**<br> **Novagraaf International S.A.**<br> **25, avenue du Pailly**<br> **1220 Les Avanchets - Geneva (CH)** |

(54) **Ultrasonic diagnostic apparatus and ultrasonic diagnostic method**

(57)     In an ultrasonic diagnostic apparatus (100) capable of generating an ultrasound image equivalent to one generated by combining images shot from various angles, without causing problems concerning an increased number of hardware pieces and a decreased frame rate, a pulsar (12) generates pulse signals for emitting ultrasound based on trigger signals received from a control unit (11), and outputs such signals to a group of transducers (1) via an amplifier (13). The group of transducers (1) receives ultrasonic echoes from a scatterer. A buffer (17) stores the reflected signals received via a delayer (14) and an A/D converter (16). A reflection angle detection unit (18) specifies a reflection angle and a reflection strength pattern of the reflected signals, and a weighting/addition unit (15) performs weighing based on such reflection strength pattern. A detection unit (19) performs detection, and a display unit (20) displays an ultrasound image and the reflection angle.

FIG. 3

EP 1 439 402 A1

**Description**

**BACKGROUND OF THE INVENTION**

**(1) Field of the Invention**

The present invention relates to an ultrasonic diagnostic apparatus that displays an ultrasound image on the basis of an echo signal obtained by transmitting and receiving ultrasound.

**(2) Description of the Related Art**

Ultrasonic diagnostic apparatuses have been widely used as indispensable apparatuses in such a field as clinical medicine, since they are capable of obtaining a two-dimensional (2D) image of soft tissues of a living body without invasion as well as offering a high level of safety to such living body to be examined.

In recent years, ultrasonic diagnostic apparatuses have been required to be capable of quantitatively calculating the size and cross section of a lesion and the like, since changes in their values are used to evaluate the progression and treatment process of the lesion. Therefore, it is also imperative for ultrasonic diagnostic apparatuses to be capable of extracting the contour and boundary of the lesion which are used to calculate its size and cross section.

The clearer an image is, the more precisely a contour and a boundary are extracted. An ultrasonic diagnostic apparatus receives ultrasonic echoes reflected back from the boundary surface of a scatterer, and generates an ultrasound image based on such reflected echoes. Therefore, if the boundary surface of such scatterer is inclined, the strength of ultrasonic echoes to be received at the points from which the ultrasound was transmitted is reduced. Because of this reason, it is impossible for the existing ultrasonic diagnostic apparatus to reproduce the distribution of actual signal strengths, and therefore to generate a desirable ultrasound image. In order to solve this problem, an existing ultrasonic diagnostic apparatus combines images shot from various angles, so as to generate one ultrasound image.

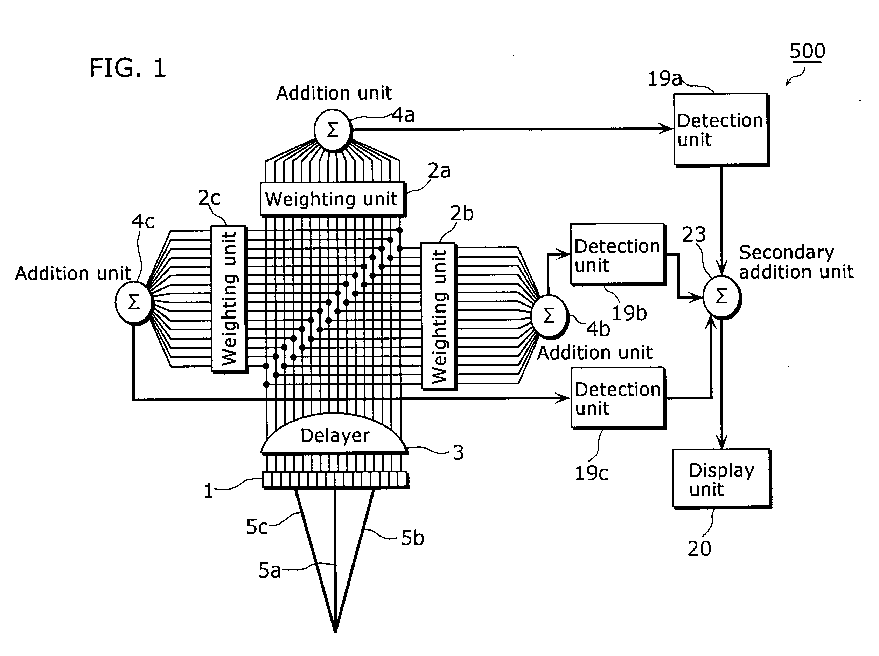

FIG. 1 is a block diagram showing a functional configuration of an existing ultrasonic diagnostic apparatus 500 that combines ultrasound images shot from various angles (Refer to Japanese Laid-Open Patent application No. 5-115479 for example).

In this ultrasonic diagnostic apparatus 500, a group of transducers 1 where plural transducers are arranged receives ultrasonic echoes, and converts them into electric signals, which are then delayed by a delayer 3. Then, such delayed signals are weighted by weighting units 2a, 2b, and 2c in respective systems (three systems are shown in FIG. 1), and the weighted electric signals are added by addition units 4a, 4b and 4c in the respective systems. Accordingly, processing i s performed based on three received beams 5a, 5b, and 5c, in appearance, so as to generate an ultrasound image. After this, the electric signals are detected by detection units 19a, 19b, and 19c in the respective systems, and these detected electric signals are combined in a secondary addition unit 23. Finally, a display unit 20 generates and displays an ultrasound image based on such combined signals.

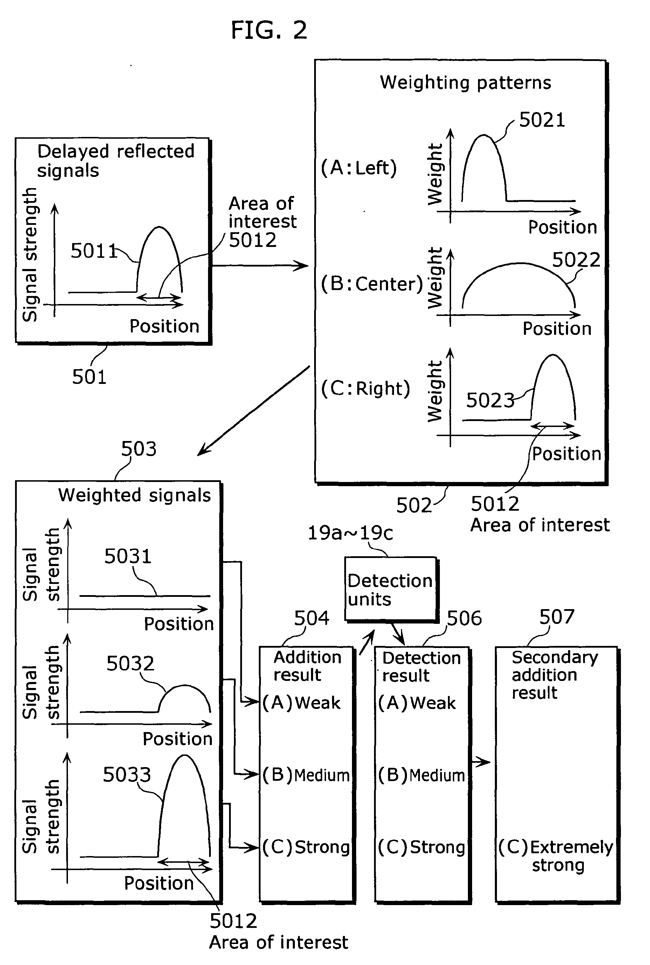

FIG. 2 is a block diagram showing an outline of the processing performed by the existing ultrasonic diagnostic apparatus 500. First, when the group of transducers 1 receives a group of reflected signals, the delayer 3 delays such received reflected signals. The strength of the resulting reflected signals 501 is strong in the right side. Subsequently, the weighting units 2a, 2b, and 2c weight the reflected signals 501 according to a weighting pattern 502 (a leftward weighting pattern 5021, a center weighting pattern 5022, and a rightward weighting pattern 5023). The weighting according to these three patterns corresponds to the reception of the received beams 5c, 5a, and 5b in FIG. 1. By weighting the reflected signals 501 according to the respective weighting patterns, a group of weighted electric signals 503 is obtained.

As FIG. 2 shows, a group of signals which has been weighted according to the leftward weighting pattern 5021 includes no signal components as a group of signals 5031. A group of signals which has been weighted according to the center weighting pattern 5022 is weak in signal strength as a group of signals 5032. And a group of signals which has been weighted according to the rightward weighting pattern 5023 is enhanced in signal strength as a group of signals 5033. When the addition units 4a, 4b, and 4c add these signals, an addition result 504 indicates signal components which are strong rightward. Furthermore, when the detection units 19a~19c detect the resulting signals, signals to be obtained are ones whose rightward signal components are strong. Then, the secondary addition unit 23 adds a detection result 506, and signals whose rightward signal components are further stronger are obtained as a secondary addition result 507.

As described above, even when most of the ultrasonic echoes are strongly reflected back from a rightward direction, the existing ultrasonic diagnostic apparatus is capable of reliably receiving such reflected ultrasound echoes and generating an ultrasound image.

However, in order to generate an ultrasound image based on ultrasonic echoes from various directions, either of the following two methods is required: combining plural images which are shot by changing angles at which ultrasonic echoes should be received; and combining plural images which are simultaneously shot from various angles. However, the former method has the problem that a frame rate decreases because of an increased number of times images are shot, whereas the latter method has the problem that more pieces of hardware are required in order to receive reflected signals from various directions all at once.

The existing ultrasonic diagnostic apparatus 500 solves the former problem about a decreased frame rate, but cannot solve the latter problem about an increased number of required hardware pieces.

**SUMMARY OF THE INVENTION**

The present invention has been conceived in view of the above problems, and it is an object of the present invention to provide an ultrasonic diagnostic apparatus capable of generating an ultrasound image which is equivalent in quality to an ultrasound image generated by combining plural images shot from various angles, without causing the above problems about an increased number of hardware pieces and a decreased frame rate.

In order to achieve the above object, the ultrasonic diagnostic apparatus according to the present invention may be an ultrasonic diagnostic apparatus, comprising: an ultrasound transmitting/receiving unit in which a plurality of transducers are arranged, each of the transducers transmitting ultrasound into an object to be examined, receiving the ultrasound reflected back from inside said object, and converting the reflected ultrasound into an electric signal; a feature detection unit operable to detect a feature of the reflected ultrasound based on the electric signal converted by each of the transducers; a weighting unit operable to weight the electric signal of each of the transducers in a specified manner, depending on the detected feature; and an image generation unit operable to generate an ultrasound image based on the weighted electric signal of each of the transducers. Moreover, in order to achieve the above object, in the above ultrasonic diagnostic apparatus, the feature detection unit may compare signal strengths of the respective electric signals of the transducers, and determine a reflection angle as the feature of the ultrasound, based on a position of one of the transducers specified by said comparison and on a position of a predetermined reception focal point.

The above configuration enables reflected signals to be weighted by hardware in a single system based on the reflection strength of ultrasonic echoes. Accordingly, it becomes possible to perform weighting with a simpler hardware configuration for performing weighting as well as to circumvent the problem about a decreased frame rate.

Note that, in order to achieve the above object, it is possible for the present invention to be embodied as an ultrasonic diagnostic method which includes, as its steps, the characteristic constitute elements of the above ultrasonic diagnostic apparatus, and as a program which includes all of such steps. Also, such program can not only be stored in a ROM and the like equipped to the ultrasonic diagnostic apparatus, but also be distributed via recording media such as CD-ROM, and transmission media such as a communication network.

As described above, since the ultrasonic diagnostic apparatus according to the present invention assigns weights to reflected signals in an appropriate manner according to a reflection angle of ultrasonic echoes. Accordingly, it is not necessary to combine images even when ultrasonic echoes are strongly reflected in a slanting direction, and therefore it becomes possible to simplify an existing hardware configuration required to perform weighting and secondary addition for a plural number of times. Therefore, the present invention is significantly effective at reducing the number of hardware pieces related to transducers which are arranged in a three-dimensional manner. Moreover, the present invention makes it possible for a reflection angle to be visualized as information about tissues of an examined object in the direction of the scatterer.

Furthermore, the present invention is capable of obtaining, from a single received beam, an image in accordance with the strength of reflected signals, without needing to generate an ultrasound image from plural received beams passing through the same point as required in an existing technique. Accordingly, it is possible to circumvent the problem about a decreased frame rate attributable to receiving beams for a number of times and the problem about an increased number of hardware pieces attributable to receiving beams from a number of angles.

For further information about the technical background to this application, Japanese Paten application No. 2003-008749 filed on January 16, 2003 is incorporated herein by reference.

**BRIEF DESCRIPTION OF THE DRAWINGS**

These and other objects, advantages and features of the invention will become apparent from the following description thereof taken in conjunction with the accompanying drawings that illustrate a specific embodiment of the invention. In the Drawings:

FIG. 1 is a block diagram showing a functional configuration of an existing ultrasonic diagnostic apparatus that combines ultrasound images shot from various angles;
FIG. 2 is a block diagram showing an outline of processing performed by the existing ultrasonic diagnostic apparatus;

FIG. 3 is a block diagram showing a functional configuration of an ultrasonic diagnostic apparatus according to the present invention;

FIG. 4 is a diagram showing an example distribution of strengths of ultrasonic echoes reflected back from a scatterer;

FIGS. 5A~5C are diagrams showing examples of weighting patterns to be specified by a weighting unit;

FIG. 6 is a diagram showing an example method of detecting reflection angles in a reflection angle detection unit;

FIG. 7 is a block diagram showing an outline of processing performed in the ultrasonic diagnostic apparatus according to the present invention;

FIG. 8 is a flowchart showing a flow of entire processing performed in the ultrasonic diagnostic apparatus according to the present invention;

FIG. 9 is a flowchart showing, in detail, an example of the reflection angle detection processing in FIG. 8; and

FIG. 10 is a flowchart showing, in detail, an example of the weighting/addition processing in FIG. 8.

## DESCRIPTION OF THE PREFERRED EMBODIMENT

The following explains the preferred embodiment according to the present invention with reference to the figures.

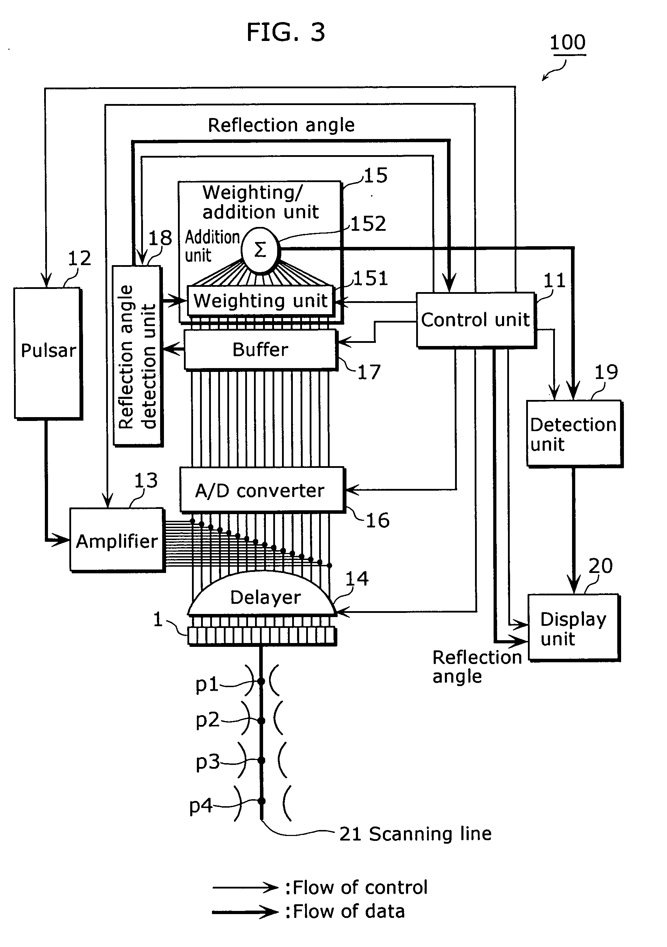

FIG. 3 is a block diagram showing a functional configuration of an ultrasonic diagnostic apparatus 100 according to the present embodiment. As FIG. 3 shows, the ultrasonic diagnostic apparatus 100 is an apparatus that generates an ultrasound image by taking into account angles at which ultrasonic echoes are reflected back from a tissue and the like of an object to be examined (to be also referred to as "scatterer" hereinafter). Such ultrasonic diagnostic apparatus 100 is comprised of a control unit 11, a pulsar 12, an amplifier 13, the group of transducers 1, a delayer 14, an A/D converter 16, a buffer 17, a weighting/addition unit 15, a reflection angle detection unit 18, a detection unit 19, and a display unit 20.

The control unit 11, which is intended for exercising an overall control of the ultrasonic diagnostic apparatus 100, is equipped with a CPU, a ROM, a RAM, and the like. In particular, the control unit 11 controls timings (e.g. synchronization) at which each unit in the ultrasonic diagnostic apparatus 100 performs processing. Moreover, the control unit 11 outputs trigger signals for controlling timings at which pulse signals generated by the pulsar 12 are outputted. Also, the control unit 11 receives a reflection angle detected by the reflection angle detection unit 18, and notifies the display unit 20 of the received reflection angle. Furthermore, the control unit 11 outputs, to the delayer 14, delay control signals used for performing focus processing.

The pulsar 12 generates pulse signals according to the trigger signals outputted from the control unit 11, and outputs such pulse signals to the amplifier 13. In this case, taking into account that the number of transducers is "T" ("T" is an arbitrary natural number, which is generally 32, 64, and 128), the control unit 11 outputs, to the delayer 14, delay control signals that allow ultrasonic echoes to be focused on at least one focal point which is located at a certain depth inside an object to be examined and to which ultrasound shall be transmitted (to be referred to as "transmission focal points" hereinafter). Then the control unit 11 determines timings of outputting the trigger signals.

The amplifier 13 amplifies the pulse signals generated by the pulsar 12, and the resultant is inputted to the group of transducers 1. The first ~ "T"th transducers in the group of transducers 1 emit ultrasound focusing on a predetermined focal point (e.g. one of or some of the positions indicated by p1~ p4 in FIG. 3). Here, transmission beams shall be formed in a horizontal direction with respect to a scanning line 21 which runs vertically to the direction in which the group of transducers 1 is laid out.

Note that ultrasound transmitted into the examined object is reflected at positions "pm"s at the depth of "m" ("m"=1, 2, ... ) one by one. The group of transducers 1 receives such reflected ultrasonic echoes, and converts them into electric signals. The converted electric signals (to be referred to as "reflected signals" hereinafter) are inputted to the delayer 14 which corresponds to each transducer in the group of transducers 1.

The delayer 14 delays the reflected signals so that the focal point a t which the reflected signals should be received (to be referred to as "reception focal point(s)" hereinafter) shifts to p1→p2→⋯→p4 sequentially with the elapse of time (processing so-called "dynamic focus"), according to an instruction from the control unit 11. Then, the delayer 14 transmits the delayed signals to the A/D converter 16.

The A/D converter 16 performs A/D conversion on the reflected signals sent by the delayer 14, and outputs the resulting signals one by one to the buffer 17 and the reflection angle detection unit 18. Here, the buffer 17 is a storage device made up of a RAM and the like for storing data indicating reflected signals corresponding to all pixels on the display unit 20.

The weighting/addition unit 15 is intended for weighting and adding the reflected signals stored in the buffer 17, and is made up of a weighting unit 151 and an addition unit 152.

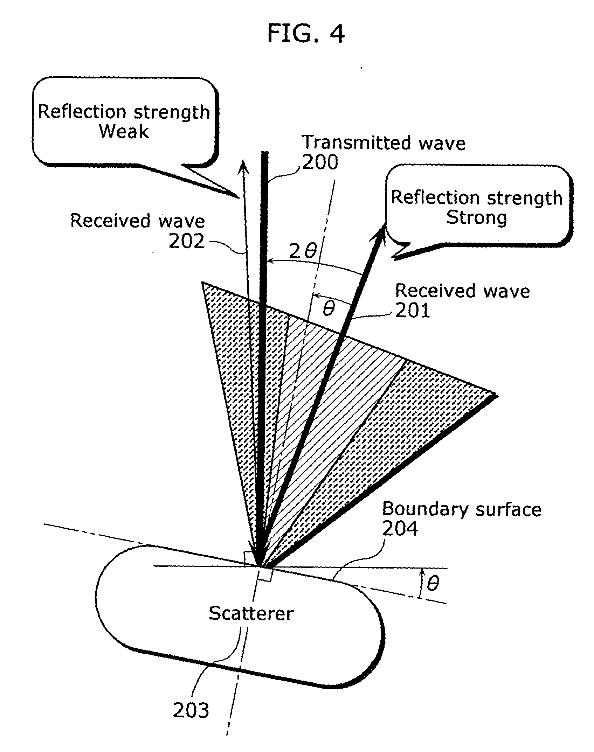

Referring to FIG. 4, an explanation is given here of the concept of weighting performed by the weighting/addition unit 15.

FIG. 4 is a diagram showing an example distribution of strengths of ultrasonic echoes reflected back from the scatterer (to be referred to as "reflection strengths" hereinafter). Generally, ultrasound is reflected at a boundary surface 204 of a scatterer 203 (e.g. a tissue in the examine object). However, when the boundary surface 204 is inclined at an angle of θ, meaning that the boundary surface 204 is not positioned vertically to the direction in which the ultrasound is transmitted, such ultrasound is strongly reflected toward a direction different from the one in which it was transmitted (in FIG. 4, such direction is one reached by inclining the boundary surface 204 by -2θ anticlockwise). Thus, when ultrasound is strongly reflected toward a direction different from the one in which it was transmitted, the weighting unit 151 performs weighting in a predetermined manner, depending on the strength of such reflected ultrasound. By performing weighting in the above manner, it is possible to generate a sharper ultrasound image.

The weighting unit 151 is capable of reading out, from the buffer 17, the data indicating the reflected signals, so as to perform weighting on the read-out data according to an instruction from the reflection angle detection unit 18. For example, the weighting unit 151 selects one of the pre-registered weighting patterns based on the signal strength pattern of a group of reflected signals specified by the reflection angle detection unit 18 to be explained later, and performs weighting according to such selected weighting pattern. This weighting unit 151 is made up of a plurality of amplifiers (not illustrated in the diagram), which perform gain control on each data under the instructions from the reflection angle detection unit 18 and the control unit 11.

Note that the above method is not an exclusive method of weighting reflected signals, and therefore that weighting may also be performed according to an equation.

Here, an explanation is given of an example method of performing weighting according to an equation. Letting that "h(t)" is the strength of a signal to be inputted from a transducer t (t: $1 \leqq t \leqq T$, T: tonal number of transducers) to the reflection angle detection unit 18, the weight "w(t)" that the weighting unit 151 assigns to an electric signal corresponding to the transducer t can be represented by the use of the following equation (1):

$$w(t) = \frac{s(t)}{\sum_{k=1}^{T} s(k)} \text{ where, } s(t) = \frac{h(t) - \min_{1 \leqq j \leqq T}(h(j))}{\max_{1 \leqq i \leqq T}(h(i)) - \min_{i \leqq j \leqq T}(h(j))} \quad (1)$$

In the above equation (1), the weight w(t) of each transducer is represented as a value which is obtained by determining a ratio (s(t)) of the difference between the reflection strength of each transducer t and the minimum reflection strength value with respect to the variations of the reflection strengths (i.e. the difference between the maximum and minimum reflection strength values) and by further normalizing each s(t). In the above equation (1), "max" and "min" are functions which respectively indicate the maximum value and the minimum value of the reflection strengths when i, j, or k changes, where $1 \leqq i$, j or $k \leqq T$.

Such weighting unit 151 is capable of dynamically changing weighting patterns. Accordingly, even when ultrasonic echoes are reflected in various directions from each position on the scanning line, it is possible for the weighting unit 151 to perform weighting according to a weighting pattern appropriate to the respective directions. Subsequently, the addition unit 152 performs addition on each weighted reflected signal outputted by the weighting unit 151 on a pixel-by-pixel basis in the display unit 20.

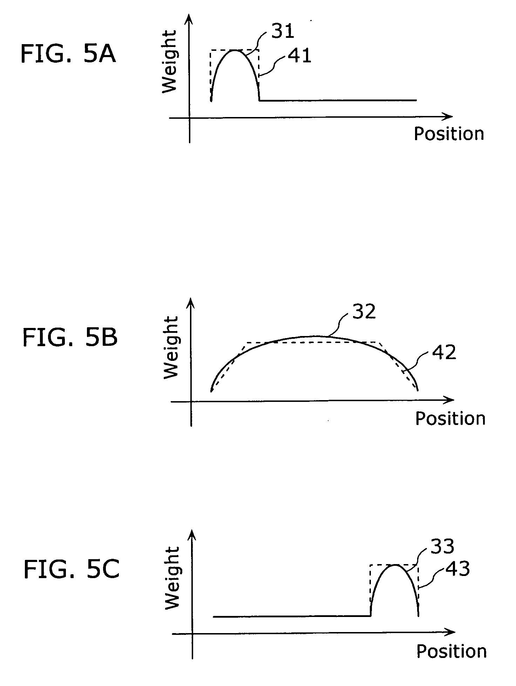

FIGs. 5A~5C are diagrams showing examples of the weighting patterns to be specified by the weighting unit 151. FIG. 5A shows the weighting pattern 31 to be used when a group of signals with strong signal strength appears in the left side of the group of transducers 1. FIG. 5B shows the weighting pattern 32 to be used when a group of signals with strong signal strength appears in the center of the group of transducers 1. FIG. 5C shows the weighting pattern 33 to be used when a group of signals with strong signal strength appears in the right side of the group of transducers 1.

Note that, as indicated by broken lines in FIGs. 5A~5C, more simplified weighting patterns 41~43 may also be used in the respective cases.

The reflection angle detection unit 18 determines a reflection angle of the ultrasonic echoes based on the values of the respective reflected signals which are outputted from the A/D converter 16 and which are obtained from the respective transducers 1~T. Furthermore, the reflection angle detection unit 18 specifies a signal strength pattern of the reflected signals, and sends the specified signal strength pattern to the weighting unit 151. For example, the reflection angle detection unit 18 detects, from among the reflected signals obtained via the group of transducers 1, a group of signals with strong signal strength, and specifies a signal strength pattern of such signals. Moreover, the reflection angle detection unit 18 determines a reflection angle on the basis of the position of a transducer that corresponds to the reflected signal with the strongest signal strength out of the group of reflected signals (A detailed explanation is given later of a method of detecting a "reflection angle").

Note that the present invention is not limited to the above-explained weighting pattern specification method, and therefore that another method may also be used. An example is pattern matching utilizing a neural network and a method that simply limits a section in which weighting should be performed.

The detection unit 19 detects the reflected signals obtained in the weighting/addition unit 15, and outputs the resultant to the display unit 20. The display unit 20 generates an ultrasound image on the basis of the reflected signals outputted from the detection unit 19, and displays the image on the CRT or the like. Furthermore, the display unit 20 displays, as the inclination of the boundary surface 204, the value of the reflection angle itself sent by the control unit 11 or such reflection angle. Accordingly, the user of the ultrasonic diagnostic apparatus 100 can know the inclination of the examined object. Note that possible methods of representing or presenting such inclination include a method of representing the inclination as color information and a method of presenting the inclination as a numeric value, but the present invention is not limited to these methods.

Also note that an ultrasonic diagnostic apparatus is normally required to take into account an anti-noise measure and signal dampening attributable to propagated distance. However, such details are not directly related to the features of the present invention, and therefore an explanation of them is omitted here.

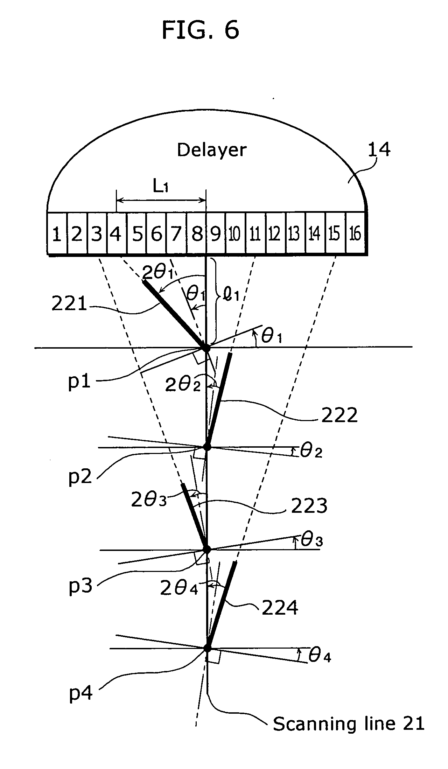

FIG. 6 is a diagram showing an example method of detecting reflection angles in the reflection angle detection unit 18. As FIG. 6 shows, the direction on the scanning line in which the ultrasonic echo with the strongest signal reception strength shifts to a direction 221→ a direction 222→ a direction 223→ a direction 224, along with the shift of the reception focal points to positions p1→p2→···→p4.

Here, let us focus on the reception focal point p1, and give an explanation of a method of detecting a reflection angle on the basis of the ultrasonic echo reflected back from such position p1. Generally, the following values are known beforehand (i.e. the following values are under the control of the control unit 11): ( i ) the timing at which ultrasound is transmitted; ( ii ) the timing at which ultrasonic echoes are received; and ( iii ) the transmission speed at which the ultrasound is transmitted inside the examined object. Therefore, based on these values ( i )~ ( iii ), it is possible to determine a vertical distance l1 from the bottom of the group of transducers 1 to the position p1 as well as a distance L1 from the scanning line 21 to the transducer that receives the ultrasonic echo with the strongest reflection strength, and to determine θ1 from l1 and L1. Note that θ2~θ4 at p2~p4 shall also be determined in this manner.

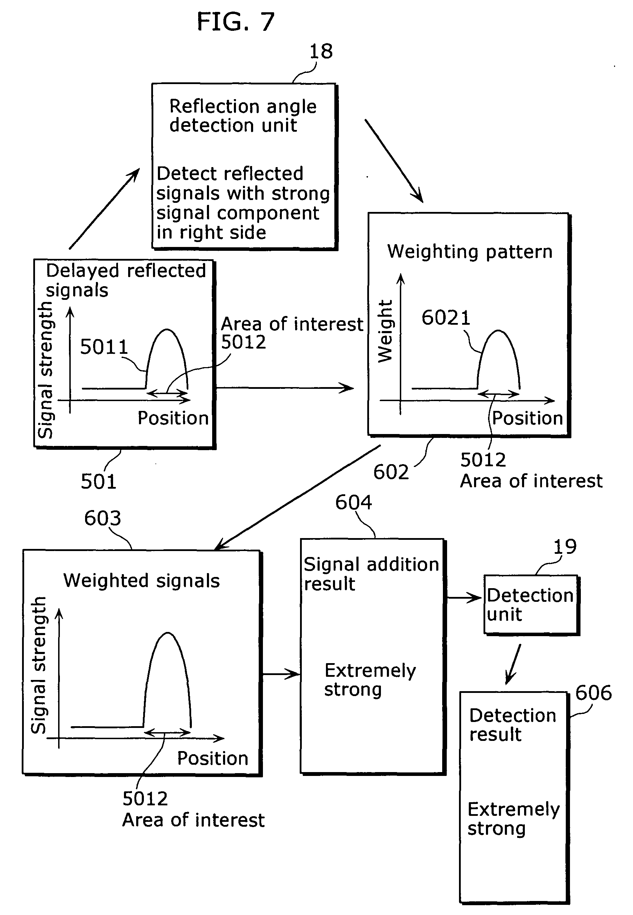

Next, referring to FIGs. 7~10, an explanation is given of the operation of the ultrasonic diagnostic apparatus 100 with the above configuration.

FIG. 7 is a block diagram showing an outline of the processing performed in the ultrasonic diagnostic apparatus 100.

First, when the A/D converted reflected signals are stored into the buffer 17 (501), the reflection angle detection unit 18 notifies the weighting/addition unit 15 that the reflection strength in the right side of the group of transducers 1 is strong, based on such reflected signals. Then, the reflection angle detection unit 18 detects a reflection angle, and notifies the weighting/addition unit 1 of the detected reflection angle. Here, an area of interest 5012 shall be the right side of the group of transducers 1 indicating a strong reflection strength. In this case, the weighting/addition unit 15 selects a weighting pattern 6021 with which weighting is performed in a specified manner, performs weighting on the area of interest 5012 according to such selected weighting pattern 6021 (603), and adds up the reflected signals (604). As a result, the result of adding the reflected signals in the area of interest 5012 as well as the detection result in the detection unit 19 will be further enhanced (in FIG. 7, such enhanced results are indicated as "Extremely strong").

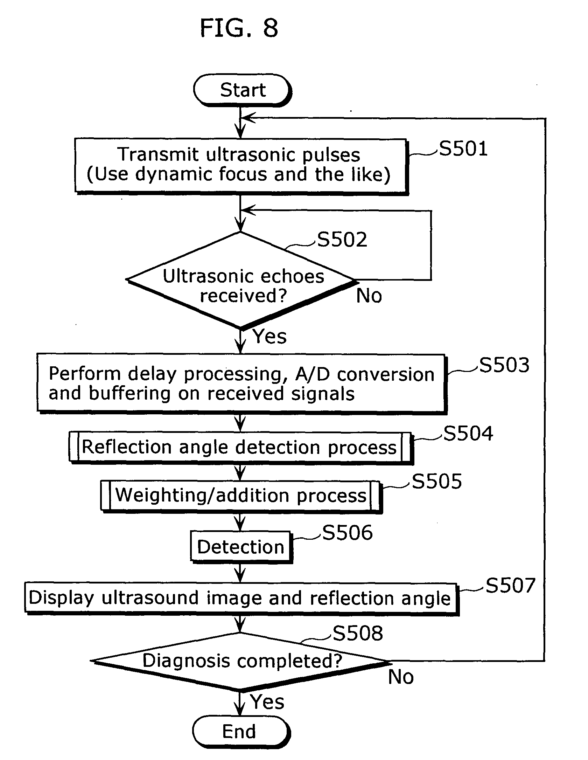

FIG. 8 is a flowchart showing the flow of the entire processing performed in the ultrasonic diagnostic apparatus 100.

First, the pulsar 12 receives trigger signals from the control unit 11, generates pulse signals for emitting ultrasound based on such trigger signals, and outputs the generated pulse signals to the group of transducers 1 via the amplifier 13 (S501). In this case, the delayer 14 generates the pulse signals so that ultrasonic pulses can be focused on at least one transmission focal point located at a certain depth inside the examined object according to delay control signals sent by the control unit 11.

Next, when the group of transducers 1 receives ultrasonic echoes reflected back from the scatterer (Yes in S502), the reflected signals are stored in the buffer 17 via the delayer 14 and the A/D converter 16 (S503).

Subsequently, the reflection angle detection unit 18 specifies a reflection angle and the signal strength pattern of the reflected signals (S504), and the weighting/addition unit 15 performs weighting and addition processing accordingly (S505).

Then, the detection unit 19 detects the reflected signals (S506), and the display unit 20 displays a generated ultrasound image and the reflection angle (S507). The above steps are repeated until the diagnosis completes (S501~S508).

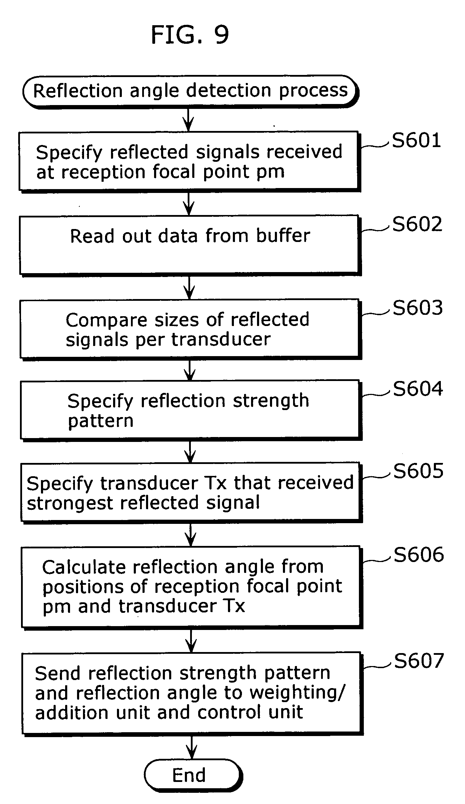

FIG. 9 is a flowchart showing, in detail, an example of the reflection angle detection process (S503) in FIG.8.

First, the reflection angle detection unit 18 specifies reflected signals received at the reception focal point pm, upon the instruction of the control unit 11 (S601), and reads out necessary data from the buffer 17 (S602).

Next, the reflection angle detection unit 18 compares the sizes of such received reflected signals on a transducer-by-transducer basis (S603), and specifies a weighting pattern based on the signal strength pattern of the reflected signals (S604). Furthermore, the reflection angle detection unit 18 specifies a transducer Tx that has received the reflected signal with the strongest signal strength (S605).

Subsequently, the reflection angle detection unit 18 determines a reflection angle from the position of the reception focal point pm and the position of the transducer Tx (S606). Then, the reflection angle detection unit 18 sends the determined reflection angle to the control unit 11 and the weighting/addition unit 15 (S607).

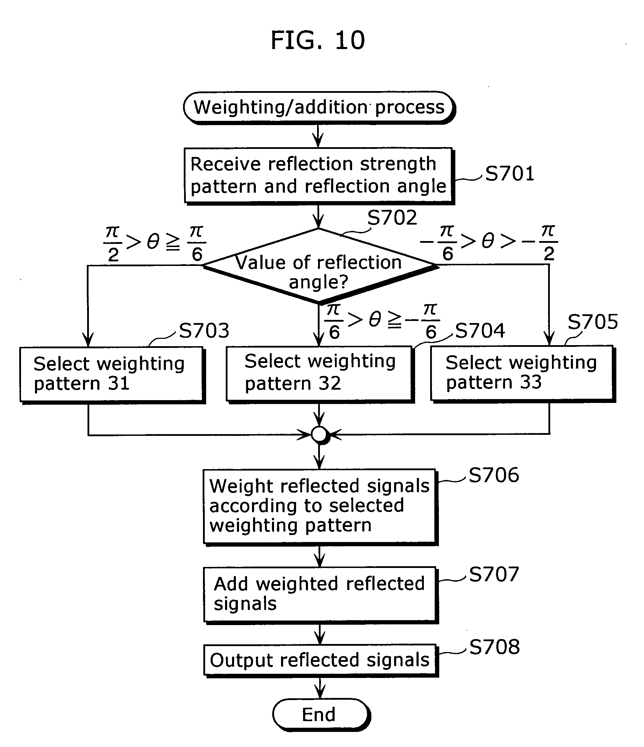

FIG. 10 is a flowchart showing, in detail, an example of the weighting/addition process (S504) in FIG. 8.

First, the weighting unit 151 receives the reflection strength pattern and the reflection angle from the reflection angle detection unit 18 (S701). On the basis of such reflection angle (S702), the weighting unit 151 selects one weighting pattern (e.g. one of the weighting patterns 31~33) (S703~S705).

Next, the addition unit 152 weights the reflected signals according to the selected weighting pattern (S706), and adds the resulting signals (S707).

Then, the addition unit 152 outputs the added reflected signals to the detection unit 19 (S708).

As described above, the ultrasonic diagnostic apparatus according to the present invention specifies a reflection angle on the basis of the strength of ultrasonic echoes and weights reflected signals according to such reflection angle. Accordingly, with a hardware configuration simpler than that of an existing ultrasonic diagnostic apparatus, it becomes possible to generate an ultrasound image which is equivalent in quality to an ultrasound image generated by such existing ultrasonic diagnostic apparatus.

Note that the above-explained embodiment presents an example in which the weighting unit 151 specifies a weighting pattern, but it is also possible that the reflection angle detection unit 18 specifies a weighting pattern instead.

Also, the above-explained embodiment presents an example in which a method of selecting a weighting pattern based on the reflection angle and a method using the equation (1) are used as the methods of determining a weighting pattern. However, the present invention is not limited to these methods, and therefore a weighting pattern may also be determined, for example, by using the learning algorithm in the neural network as well as by employing pattern matching.

Furthermore, the above-explained embodiment presents an example in which transducers are arranged in a one-dimensional array, but the present invention is not limited to such arrangement. Therefore, the present invention is also applicable to a group of transducers in two-dimensional or three-dimensional array.

Moreover, the ultrasonic diagnostic apparatus may be implemented as any one of an analog circuit, a digital circuit, and software.

**Claims**

1. An ultrasonic diagnostic apparatus, comprising:

   an ultrasound transmitting/receiving unit in which a plurality of transducers are arranged, each of the transducers transmitting ultrasound into an object to be examined, receiving the ultrasound reflected back from inside said object, and converting the reflected ultrasound into an electric signal;
   a feature detection unit operable to detect a feature of the reflected ultrasound based on the electric signal converted by each of the transducers;
   a weighting unit operable to weight the electric signal of each of the transducers in a specified manner, depending on the detected feature; and
   an image generation unit operable to generate an ultrasound image based on the weighted electric signal of each of the transducers.

2. The ultrasonic diagnostic apparatus according to Claim 1,
   wherein the weighting unit modifies the weighting and further weights the electric signal of each of the transducers in the modified manner, depending on the detected feature, and
   the image generation unit generates the ultrasound image based on the electric signal of each of the transducers which has been weighted in said modified manner.

3. The ultrasonic diagnostic apparatus according to Claim 1,
   wherein the feature detection unit compares signal strengths of the respective electric signals of the transducers, and determines a reflection angle as the feature of the ultrasound, based on a position of one of the transducers specified by said comparison and on a position of a predetermined reception focal point.

**4.** The ultrasonic diagnostic apparatus according to Claim 3, further comprising a storage unit operable to store a plurality of weighting patterns,
wherein the weighting unit selects one of the weighting patterns stored in the storage unit based on the reflection angle detected by the feature detection unit, and weights the electric signal of each of the transducers according to said selected weighting pattern.

**5.** The ultrasonic diagnostic apparatus according to Claim 3, further comprising a display unit operable to display the reflection angle detected by the feature detection unit.

**6.** The ultrasonic diagnostic apparatus according to Claim 1,
wherein the feature detection unit measures a signal strength of the electric signal of each of the transducers; and uses said signal strength as the feature of the ultrasound.

**7.** The ultrasonic diagnostic apparatus according to Claim 6,
wherein the weighting unit determines a weight w(t) for the electric signal corresponding to an arbitrary "t"th transducer using the following equation, letting that h(t) is the measured signal strength of said "t"th transducer:

$$w(t) = \frac{s(t)}{\sum_{k=1}^{T} s(k)} \text{ where, } s(t) = \frac{h(t) - \min_{1 \leq j \leq T}(h(j))}{\max_{1 \leq i \leq T}(h(i)) - \min_{i \leq j \leq T}(h(j))}$$

**8.** An ultrasonic diagnostic method comprising:

an ultrasound transmitting/receiving step of transmitting ultrasound into an object to be examined, receiving the ultrasound reflected back from inside said object, and converting the reflected ultrasound into an electric signal using each of a plurality of transducers which are arranged;
a feature detection step of detecting a feature of the reflected ultrasound based on the electric signal converted by each of the transducers;
a weighting step of weighting the electric signal of each of the transducers in a specified manner, depending on the detected feature; and
an image generation step of generating an ultrasound image based on the weighted electric signal of each of the transducers.

**9.** The ultrasonic diagnostic method according to Claim 8,
wherein in the weighting step, the weighting is modified and the electric signal of each of the transducers is further weighted in the modified manner, depending on the detected feature, and
in the image generation step, the ultrasound image is generated based on the electric signal of each of the transducers which has been weighted in said modified manner.

**10.** The ultrasonic diagnostic method according to Claim 8,
wherein in the feature detection step, signal strengths of the respective electric signals of the transducers are compared, and a reflection angle is determined as the feature of the ultrasound, based on a position of one of the transducers specified by said comparison and on a position of a predetermined reception focal point.

**11.** A program for an ultrasonic diagnostic apparatus, the program causing a computer to execute the following steps:

an ultrasound transmitting/receiving step of transmitting ultrasound into an object to be examined, receiving the ultrasound reflected back from inside said object, and converting the reflected ultrasound into an electric signal using each of a plurality of transducers which are arranged;
a feature detection step of detecting a feature of the reflected ultrasound based on the electric signal converted by each of the transducers;

a weighting step of weighting the electric signal of each of the transducers in a specified manner, depending on the detected feature; and
an image generation step of generating an ultrasound image based on the weighted electric signal of each of the transducers.

12. The program according to Claim 11,
wherein in the weighting step, the weighting is modified and the electric signal of each of the transducers is further weighted in the modified manner, depending on the detected feature, and
in the image generation step, the ultrasound image is generated based on the electric signal of each of the transducers which has been weighted in said modified manner.

13. The program according to Claim 11,
wherein in the feature detection step, signal strengths of the respective electric signals of the transducers are compared, and a reflection angle is determined as the feature of the ultrasound, based on a position of one of the transducers specified by said comparison and on a position of a predetermined reception focal point.

FIG. 1

500

- Detection unit — 19a
- Secondary addition unit — 23
- Display unit — 20
- Detection unit — 19b
- Detection unit — 19c
- Addition unit — 4a
- Addition unit — 4c
- Addition unit — 4b
- Weighting unit — 2a
- Weighting unit — 2b
- Weighting unit — 2c
- Delayer — 3
- 1, 5a, 5b, 5c

# FIG. 2

# FIG. 3

<u>100</u>

Reflection angle

Weighting/
addition unit — 15

Addition
unit — 152

Σ

Weighting unit — 151

Control unit — 11

Reflection angle detection unit — 18

Pulsar — 12

Buffer — 17

Detection
unit — 19

Amplifier — 13

A/D converter — 16

Delayer — 14

1

Display
unit — 20

Reflection
angle

p1

p2

p3

p4

21 Scanning line

——→ : Flow of control
━━➤ : Flow of data

EP 1 439 402 A1

FIG. 4

13

FIG. 5A

Weight

31
41

Position

FIG. 5B

Weight

32
42

Position

FIG. 5C

Weight

33
43

Position

# FIG. 6

## FIG. 7

18

Reflection angle
detection unit

Detect reflected
signals with strong
signal component
in right side

Weighting pattern

Weight

6021

Position

5012
Area of interest

602

Delayed reflected
signals

Signal strength

5011

Area of interest
5012

Position

501

603

Weighted signals

Signal strength

Position

5012
Area of interest

604

Signal addition
result

Extremely
strong

19

Detection
unit

606

Detection
result

Extremely
strong

## FIG. 8

```
            ┌─────────┐
            │  Start  │
            └────┬────┘
                 ▼
    ┌────────────────────────────┐
    │   Transmit ultrasonic pulses│ ～S501
    │ (Use dynamic focus and the like)│
    └────────────┬───────────────┘
                 ▼
             ╱╲  ～S502
            ╱    ╲
           ╱      ╲
          ╱ Ultrasonic echoes ╲  No
          ╲   received?       ╱──────┐
           ╲      ╱
            ╲    ╱
             ╲╱
              │ Yes
              ▼
    ┌────────────────────────────────┐
    │ Perform delay processing, A/D conversion│ ～S503
    │ and buffering on received signals│
    └────────────┬───────────────────┘
                 ▼
    ┌────────────────────────────────┐
    │ Reflection angle detection process│ ～S504
    └────────────┬───────────────────┘
                 ▼
    ┌────────────────────────┐
    │ Weighting/addition process│ ～S505
    └────────────┬───────────┘
                 ▼
    ┌────────────┐
    │ Detection  │ ～S506
    └──────┬─────┘
           ▼
    ┌────────────────────────────────────┐
    │ Display ultrasound image and reflection angle│ ～S507
    └────────────┬───────────────────────┘
                 ▼
             ╱╲  ～S508
            ╱    ╲
    Diagnosis completed?  No
            ╲    ╱──────┐
             ╲╱
              │ Yes
              ▼
          ┌─────────┐
          │   End   │
          └─────────┘
```

# FIG. 9

```
( Reflection angle detection process )
                    │
                    ▼
┌──────────────────────────────────┐  S601
│ Specify reflected signals received│
│ at reception focal point pm       │
└──────────────────────────────────┘
                    │
                    ▼
┌──────────────────────────────────┐  S602
│      Read out data from buffer    │
└──────────────────────────────────┘
                    │
                    ▼
┌──────────────────────────────────┐  S603
│   Compare sizes of reflected      │
│   signals per transducer          │
└──────────────────────────────────┘
                    │
                    ▼
┌──────────────────────────────────┐  S604
│    Specify reflection strength    │
│    pattern                        │
└──────────────────────────────────┘
                    │
                    ▼
┌──────────────────────────────────┐  S605
│ Specify transducer Tx that received│
│ strongest reflected signal        │
└──────────────────────────────────┘
                    │
                    ▼
┌──────────────────────────────────┐  S606
│  Calculate reflection angle from  │
│  positions of reception focal point│
│  pm and transducer Tx             │
└──────────────────────────────────┘
                    │
                    ▼
┌──────────────────────────────────┐  S607
│ Send reflection strength pattern  │
│ and reflection angle to weighting/│
│ addition unit and control unit    │
└──────────────────────────────────┘
                    │
                    ▼
                 ( End )
```

## FIG. 10

Weighting/addition process

Receive reflection strength pattern and reflection angle —— S701

S702

$\dfrac{\pi}{2} > \theta \geqq \dfrac{\pi}{6}$     Value of reflection angle?     $-\dfrac{\pi}{6} > \theta > -\dfrac{\pi}{2}$

$\dfrac{\pi}{6} > \theta \geqq -\dfrac{\pi}{6}$   S704     S705

S703

Select weighting pattern 31     Select weighting pattern 32     Select weighting pattern 33

Weight reflected signals according to selected weighting pattern —— S706

Add weighted reflected signals —— S707

Output reflected signals —— S708

End

<table>
<tr><td colspan="2" style="text-align:center"><strong>European Patent Office</strong></td><td style="text-align:center"><strong>EUROPEAN SEARCH REPORT</strong></td><td style="text-align:center">Application Number<br><strong>EP 04 00 0506</strong></td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | PATENT ABSTRACTS OF JAPAN<br>vol. 0174, no. 68 (C-1102),<br>26 August 1993 (1993-08-26)<br>-& JP 05 115479 A (FUJITSU LTD),<br>14 May 1993 (1993-05-14)<br>* abstract *<br>* paragraph [0008] - paragraph [0021] *<br>* paragraph [0031] - paragraph [0034] *<br>--- | 1-4,6,<br>8-13 | G01S15/89<br>A61B8/08<br>G01N29/06 |
| X<br><br>A | US 5 269 307 A (FIFE MICHAEL J ET AL)<br>14 December 1993 (1993-12-14)<br>* column 6, line 26 - column 16, line 65;<br>figure 7 *<br>--- | 1,2,6,8,<br>9,11,12<br>3,10 | |
| X<br><br>A | US 5 111 825 A (NISHIYAMA HISASHI ET AL)<br>12 May 1992 (1992-05-12)<br>* column 2, line 38 - column 3, line 57;<br>claim 1 *<br>--- | 1,8<br><br>11 | |
| A | US 6 182 512 B1 (LORRAINE PETER WILLIAM)<br>6 February 2001 (2001-02-06)<br>* column 2, line 56 - column 5, line 43;<br>figures 1,2A,2B *<br>--- | 1,2,4,8,<br>9,11,12 | TECHNICAL FIELDS SEARCHED (Int.Cl.7)<br><br>G01S<br>A61B |
| A | US 5 601 083 A (ANDERSON FORREST)<br>11 February 1997 (1997-02-11)<br>* abstract *<br>* column 13, line 4 - column 14, line 55 *<br>----- | 1,8,11 | G01N |

The present search report has been drawn up for all claims

| Place of search | Date of completion of the search | Examiner |
|---|---|---|
| MUNICH | 31 March 2004 | Artikis, T |

**ANNEX TO THE EUROPEAN SEARCH REPORT
ON EUROPEAN PATENT APPLICATION NO.**

EP 04 00 0506

This annex lists the patent family members relating to the patent documents cited in the above-mentioned European search report.
The members are as contained in the European Patent Office EDP file on
The European Patent Office is in no way liable for these particulars which are merely given for the purpose of information.

31-03-2004

| Patent document cited in search report | | Publication date | Patent family member(s) | | Publication date |
|---|---|---|---|---|---|
| JP 05115479 | A | 14-05-1993 | NONE | | |
| US 5269307 | A | 14-12-1993 | EP | 0726731 A1 | 21-08-1996 |
| | | | JP | 7506736 T | 27-07-1995 |
| | | | WO | 9314698 A1 | 05-08-1993 |
| US 5111825 | A | 12-05-1992 | JP | 3151944 A | 28-06-1991 |
| US 6182512 | B1 | 06-02-2001 | NONE | | |
| US 5601083 | A | 11-02-1997 | US | 5235857 A | 17-08-1993 |
| | | | US | 5090245 A | 25-02-1992 |
| | | | US | 5134884 A | 04-08-1992 |
| | | | US | 4706499 A | 17-11-1987 |
| | | | US | 5027658 A | 02-07-1991 |
| | | | US | 5005418 A | 09-04-1991 |

EPO FORM P0459

For more details about this annex : see Official Journal of the European Patent Office, No. 12/82